(19)

Europäisches
Patentamt
European
Patent Office
Office européen
des brevets

(11) **EP 2 962 323 B1**

(12) **EUROPEAN PATENT SPECIFICATION**

(45) Date of publication and mention
of the grant of the patent:
**02.08.2017 Bulletin 2017/31**

(51) Int Cl.:
***H01J 35/06*** *(2006.01)*  ***H01J 35/18*** *(2006.01)*
***H01J 35/12*** *(2006.01)*

(21) Application number: **14709739.8**

(86) International application number:
**PCT/GB2014/050559**

(22) Date of filing: **25.02.2014**

(87) International publication number:
**WO 2014/132049 (04.09.2014 Gazette 2014/36)**

(54) **APPARATUS FOR THE GENERATION OF LOW-ENERGY X-RAYS**

VORRICHTUNG ZUR ERZEUGUNG VON RÖNTGENSTRAHLEN MIT GERINGER ENERGIE

APPAREIL DE GÉNÉRATION DE RAYONS X À FAIBLE ÉNERGIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.02.2013 GB 201303517**

(43) Date of publication of application:
**06.01.2016 Bulletin 2016/01**

(73) Proprietor: **Enxray Limited
Liverpool, L7 9NJ (GB)**

(72) Inventors:
• **UDALOV, Yuri
Liverpool, L7 9NJ (GB)**

• **MITKO, Sergey
Liverpool, L7 9NJ (GB)**

(74) Representative: **Pitchford, James Edward
Mathys & Squire LLP
The Shard
32 London Bridge Street
London SE1 9SG (GB)**

(56) References cited:
**EP-A1- 1 058 286        WO-A1-2005/086203
US-A1- 2007 189 459**

**Description**

**Field of the Invention**

The present invention relates to apparatus for the generation of X-rays. It is particularly applicable, but by no means limited, to low-energy X-ray generators for sterilising medical articles, pharmaceutical products, or packaging for food or drink products. Other possible applications are discussed below.

**Background to the Invention**

X-ray generators are often used in manufacturing or packaging facilities to sterilise medical articles, pharmaceutical products, or packaging for food or drink products.

In such applications, such as the sterilisation of packaging, conventionally the article to be sterilised is exposed to *X-ray* radiation produced by means of a radioactive source such as radioactive cobalt. Such radiation comprises *"hard"* X-rays, *i.e.* radiation having a high energy, measured in millions of electron volts *(eV).*

*"Hard"* X-rays are typically produced by a radioactive decay process, when nuclei undergo a transition into a different element of the periodic table, simultaneously emitting energy through electromagnetic waves. This happens in so-called *"Gamma-factories",* which utilize the decay of radioactive cobalt and emit high-energy *X-ray* photons (in this particular case called gamma-particles, although this is still *X-ray* radiation, just having a specific energy or wavelength).

Current sterilisation standards require a dosage of the order of 25 kGy (kilograys) to achieve efficient destruction of bacteria to an acceptable level. Such a dosage requires exposure of the packaging to a radioactive source for a prolonged period of time, usually several hours. For this to be practicable, such sterilisation is generally carried out in batches comprised of one or more pallet-loads of products. This is possible because the *"hard"* X-rays, by virtue of their high energy, have the ability to penetrate deep into a large stack of packages.

However, more recently, as discussed in GB 2444310 A, it has been found that low energy or *"soft"* X-rays can be better suited to the sterilisation of surfaces. *"Soft"* X-rays are characterised by being of relatively low energy, with quantum energies predominantly in the range of 5 to 20 keV. Because of their low energy, these soft X-rays have higher absorption. As a result, the efficiency of the *X-rays* in destroying bacteria on the surface is high and the total exposure that is required may be lower that when using high energy *X-rays.* The lower *X-ray* dosage is also desirable in that it reduces the risk of damaging the material being sterilised, and the softness of the *X-rays* also allows them to be used safely in a production line without risk to personnel or the need for extensive lead shielding.

Such *"soft"* X-rays can be generated using a particle accelerator (for example, an electron gun) to generate a flow of charged particles. When these particles are decelerated due to the interaction with matter - for example, when they hit a metal target - they emit electromagnetic radiation. If the initial energy of the particle beam is sufficiently high, the electromagnetic radiation is located in the *X-ray* range of the emission spectrum.

Electrons also emit *X-rays* when they change the direction of their motion, as in the case of synchrotrons (synchrotron radiation can be generated in a broad spectral range, including *X-rays).*

In the present work, we deal with the acceleration-based approach to soft *X-ray* production. The majority of *X-ray* sources operate at low-power levels (as these are medical/dental *X-ray* apparatuses, non-destructive testing **(NDT)** and luggage screening equipment). This is determined by the specific tasks of the equipment: they have to produce an *X-ray* beam which will assure the best possible image quality. Also, these sources are typically generated as a point-like source, due to the requirements of *X-ray* imaging. The best way to create such beams for imaging applications is to accelerate electrons in a vacuum and then to direct them onto a metal target.

However, vacuum-based *X-ray* tubes with heated cathodes are not well suited for long periods of heavy duty operation, as would be the case with sterilisation applications. Therefore, in the present work, we have chosen an approach to *X-ray* production based on the generation of electrons in gas-filled devices with cold cathodes, rather than in vacuum-based electron beam sources with heated filaments.

Existing *"soft"* X-ray generator systems, such as the one disclosed in GB 2444310 A, suffer from a number of disadvantages, at least in part resulting from the arrangement of the cathode and anode electrodes and the occurrence of arcing between them. Arcing between the electrodes, and the consequent erosion of the electrodes, leads to a decrease in the operational lifetime of the system. It also affects its ability to produce stable, reliable and reproducible *X-rays* homogeneously over a large cross-section of the emitter head, and to provide continuous and consistent operation.

Embodiments of the present invention therefore seek to achieve one or more of the following: (1) to increase the operational lifetime of the *X-ray* generator system; (2) to enable it to operate over a large cross-section of the *X-ray* emitter head; (3) to improve stability and reproducibility (minimizing pulse to pulse variation of energy generated) of the device; and (4) to avoid arcing and discharge instabilities which reduce the reliability of the device when used in industrial settings requiring high levels of continuous and consistent operation.

Background art is provided in EP 1 058 286 A1, which discloses a miniature *X-ray* source for intravascular treatment of lesions in body tissue, the *X-ray* source comprising a first support member and a second support member, wherein the first and second support members are joined together so as to form a cavity between them and such that they are electrically insulated from each other. A cathode and an anode are disposed inside the cavity and opposite each other. The cathode is suitably made of diamond like material, and comprises at least one pointed element.

**Summary of the Invention**

The present invention is an X-ray source as defined in Claim 1 of the appended claims. Also provided is a method as defined in Claim 15.

Thus, according to a first aspect of the invention there is provided an X-ray source for producing soft X-rays for sterilising an article, the X-ray source comprising: a cathode having an electron-emitting structure supported by a support structure, the electron-emitting structure being at least partially transparent to X-rays within a region bounded by the support structure; an anode having an X-ray emitting surface parallel to the electron-emitting structure of the cathode; and an electrically insulating spacer arranged between the anode and the cathode; wherein the electron-emitting structure of the cathode and the *X-ray* emitting surface of the anode are arranged such that, in use, the electron-emitting structure is operable to bombard the anode with electrons, causing *X-rays* to be emitted from the *X-ray* emitting surface and to pass through the cathode; and wherein the insulating spacer is arranged between the anode and the support structure of the cathode and projects beyond the support structure, across part of the anode, into the said region.

The expression "region bounded by the support structure" as used above and herein should be interpreted broadly, to encompass an arrangement in which the support structure is present on only two opposing sides of the region in question, as well as arrangements in which the support structure substantially or completely surrounds the region in question.

By virtue of the insulating spacer projecting beyond the cathode support structure, into the said region, across part of the anode, this avoids or at least mitigates the formation of places in the vicinity of the cathode and anode where the electric field strength could otherwise rise substantially. In effect, the insulating spacer "smoothes" the electric field distribution in the vicinity of the anode and cathode. This reduces the probability of electric breakdown between the cathode and anode electrodes, thereby reducing the likelihood of arcing between the electrodes, and reducing the occurrence of erosion of the electrodes. As a consequence, this increases the operational lifetime of the *X-ray* generator system, makes it more usable for continuous and consistent operation at elevated power levels, enables it to deliver a more homogeneous discharge over a large cross-section of the *X-ray* emitter head, and improves the overall stability, reliability and reproducibility of the device.

In a presently-preferred embodiment the distance of projection of the insulating spacer beyond the cathode support structure, into the said region, is about 15 mm. This has been found to give optimum results.

Preferably the width of the *X-ray* emitting surface not covered by the insulating spacer is in the range of about 3 cm to about 10 cm.

Preferably the thickness of the insulating spacer is about 2 mm.

Preferably the insulating spacer is made of a ceramic material such as alumina ($Al_2O_3$). However, other insulating materials (in particular, other ceramics) may be used instead.

Preferably the electron-emitting structure of the cathode has a grid or mesh structure. Particularly preferably the geometric transparency of the grid or mesh structure is about 70% to 80%.

According to the present invention the *X-ray* source further comprises an *X-ray* transparent window on the opposite side of the cathode from the anode, the window defining a chamber between the window and the anode; wherein the chamber contains a gas at sub-atmospheric pressure such as to allow a discharge in gas. The gas may be an inert gas such as helium or nitrogen, or may be air. Preferably a molecular sieve is provided between the gas supply and the chamber, to prevent moisture or dust etc. from entering the chamber. A vacuum pump may also be provided in communication with the chamber, to achieve and maintain sub-atmospheric pressure within the chamber.

Preferably the *X-ray* transparent window comprises Kapton™ (RTM), as this has been found to have advantageous properties (including becoming stronger when exposed to *X-rays,* whereas other materials can break down or become brittle over time).

Particularly preferably the window is formed of an electrically conductive material, or further comprises a coating formed of an electrically conductive material. This enables the window to be held at the same electric potential as the electron-emitting structure of the cathode, thereby preventing charged particles from the cathode being accelerated towards the window and damaging it. Accordingly, the window may advantageously be electrically connected to the electron-emitting structure of the cathode.

Preferably the anode is formed of a metal block, at least several millimetres in thickness.

Preferably the anode further comprises cooling means, such as one or more cooling pipes in thermal communication with the anode.

Preferably the electron-emitting structure of the cathode is at least partly formed of copper. Preferably the anode is also at least partly formed of copper. For example, it may be formed of bulk copper, or iron coated with copper. Although copper is our presently-preferred material for the cathode and anode, other materials may be used instead, provided they have characteristic emission lines in the spectral range below 10-12 keV.

The *X-ray* source may further comprise a power supply cable electrically connected to the anode.

Preferably the *X-ray* source further comprises isolation material configured to match the wave impedance of the power supply cable with the wave impedance of the anode. This advantageously reduces reflections of the voltage pulses applied to the emitter head.

Preferably the electron-emitting structure of the cathode is at ground potential.

Preferably the electron-emitting structure of the cathode is electrically connected to the cathode support structure, enabling the electron-emitting structure and the cathode support structure to be held at a common potential.

Preferably the cathode support structure is connected to, or integrally formed with, a housing structure for the *X-ray* source. The housing structure may be arranged around at least part of the anode.

Preferably the insulating spacer extends between the housing structure and the anode.

Preferably the *X-ray* source further comprises means for generating a voltage between the anode and the cathode.

Particularly preferably the means for generating a voltage comprises inductive energy storage means. With such an arrangement, rising current results in a voltage rise on the inductors, thus effectively reducing the voltage applied to any spark that may be created within the *X-ray* generator - effectively functioning as a self-damping limiter. This further improves the operational stability and longevity of the *X-ray* device.

Preferably the means for generating a voltage is configured to supply high-voltage short-duration pulses to the anode.

Preferably the *X-ray* source is configured to emit *X-rays* with quantum energies in the range of 5 keV to 20 keV, although this energy may be increased if required by a particular application.

The present disclosure also provides sterilisation apparatus comprising an *X-ray* source in accordance with the first aspect of the invention.

The present disclosure also provides a production line or a manufacturing or packaging facility comprising said sterilisation apparatus.

According to a second aspect of the invention there is provided a method of sterilising an article, the method comprising irradiating the article with *X-ray* radiation using an *X-ray* source in accordance with the first aspect of the invention.

The article being irradiated may be, for example, a medical article, a pharmaceutical product, packaging material for a food or drink product, a plastic film, a blood sample, or a foodstuff or beverage.

The present disclosure also provides an outcoupling window for an *X-ray* source, the window comprising a material that is at least partially transparent to X-rays; wherein the window is formed of an electrically conductive material or further comprises a coating formed of an electrically conductive material.

**Brief Description of the Drawings**

Embodiments of the invention will now be described, by way of example only, and with reference to the drawings in which:

Figure 1 is a schematic cross-sectional diagram of an *X-ray* generator according to an embodiment of the present invention;

Figure 2 illustrates *X-ray* transmission data for a Kapton™ window of the X-ray generator (the plotted data being *X-ray* transmission data in respect of Kapton™ polyimide film 75 $\mu$m thick);

Figure 3 is a plot of mass-energy absorption coefficient versus photon energy for typical plastic packaging material (of density 1 g cm$^{-3}$);

Figure 4 is a plot of dose efficiency as a function of photon energy;

Figure 5 illustrates the generation of *X-rays* by an electron beam impinging on a metal target;

Figures 6a, 6b and 6c provide a comparison of theoretical and experimental bremsstrahlung spectra;

Figure 7 is a plot showing the intensity of Cu K$\alpha$ characteristic radiation;

Figure 8 is a schematic diagram of production line apparatus in which a plastic film is sterilised with *X-rays;*

Figure 9 is a plot showing specific dose-rate distribution along a plastic film; and

Figure 10 illustrates representative data for the dose-area integral for a copper anode *X-ray* source, calculated for different distances and voltages.

**Detailed Description of Preferred Embodiments**

The present embodiments represent the best ways known to the applicants of putting the invention into practice. However, they are not the only ways in which this can be achieved.

*Overview of presently-preferred embodiment*

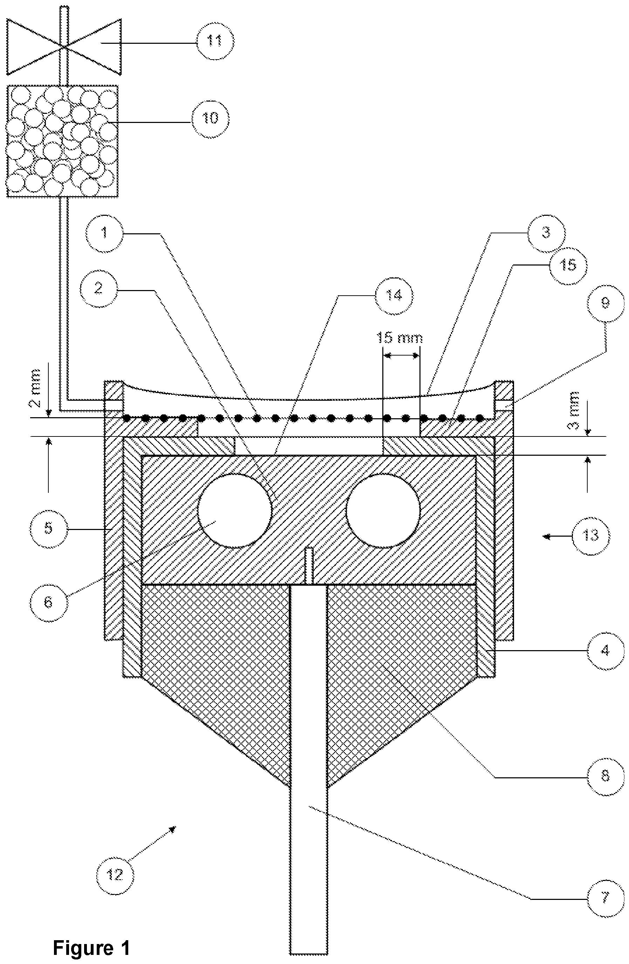

Figure 1 illustrates an *X-ray* generator 12 comprising a gas-filled flash *X-ray* tube with inductive energy storage for the sterilisation of products such as plastic medical articles. The emitter head 13 of the *X-ray* tube comprises a cold cathode 1 made of a highly transparent metal grid or mesh, and an anode 2 made of massive metal energized by high-voltage short-duration pulses. Electrons emitted by the grid cathode 1 strike the metal anode 2 and generate characteristic and bremsstrahlung *X-ray* radiation from the emission surface 14 of the anode 2. The *X-ray* radiation passes through the cathode grid 1 and irradiates the article(s) to be sterilised.

An electrically insulating (preferably ceramic) spacer 4 provides means to avoid shorting or arcing of the anode-cathode discharge gap upon application of pulsed power, thereby achieving increased operational lifetime and more stable and reproducible operation, while also creating conditions for generating an *X-ray* beam over a large area.

Furthermore, in the presently-preferred embodiment, a power supply based not on capacitive energy storage but on inductive energy storage is used.

It should be noted that the diagram in Figure 1 is not to scale. Furthermore, the measurements included in this diagram relate only to a presently-preferred embodiment, and are by way of example only; in alternative embodiments the constituent features may have different measurements. The component regions on the left side of Figure 1 predominantly mirror those on the right; for clarity each component has been labelled only once.

*Detailed description of X-ray generator*

Figure 1 illustrates an *X-ray* generator 12 according to a presently-preferred embodiment of the present invention. A homogeneous *X-ray* beam is generated from an irradiator with a large cross-section area, rather than from a point source. A wide range of shapes and dimensions of the emitter head 13 are possible. For example, it may be long and thin *(e.g.* extending with uniform cross-section normal to the plane of Figure 1), round or square, or any other shape - depending upon the requirements dictated by the shape of the target being irradiated.

As illustrated, the electrode system of the *X-ray* generator 12 comprises a cathode 1 and an anode 2. The cathode 1 has a grid or mesh electron-emitting structure (as described in more detail below; a number of different metals which possess good heat and electric conductivity may be used). The cathode 1 is shaped and configured such that *X-rays* can penetrate the structure relatively freely. In the presently-preferred embodiment the mesh of the cathode 1 has a geometric transparency of about 70% to 80% (below this range it will work less efficiently, lowering the energy yield conversion into *X-rays,* while above this range the mesh may become too fragile and break).

The anode 2 is made of a metal block at least several millimetres in thickness, which provides the possibility for enhanced cooling of the anode 2 and heat removal via cooling pipes 6. This is important for stable and continuous operation of the device in a real operating environment. The metal block may be cooled using a wide range of cooling systems employing a heat exchange. For example, a water based cooling system operating at a rate of 1 litre/second would be sufficient to dissipate 200 kW of heat energy absorbed by the metal block.

The preferred material for the electrodes 1, 2 is copper, due to the fact that copper emits a strong line of characteristic radiation Cu K - the first characteristical K emission line of copper in a low-energy (8 keV) part of the X-ray spectrum. However, it is possible to make the electrodes 1, 2 from other metals or conductive materials, of which their surface can be covered with a thin copper layer to provide similar emission properties to bulk copper. It is preferable that both cathode 1 and anode 2 consist of, or have their surface covered with, similar material in order to avoid an eventual change in the emission spectrum properties due to changes of the surface composition in the case of spattering, which can occur if the electrodes 1, 2 are composed of different materials. Copper is our presently-preferred material for the electrodes 1, 2; however, other materials may be used instead, provided they have characteristic emission lines in the spectral range below 10-12 keV.

The gap between the cathode 1 and the anode 2 is filled with gas at sub-atmospheric pressure (low or intermediate pressure). It can be a specially selected inert gas, such as helium or nitrogen, but alternatively normal air can be used to fill in the device. Gas pressure inside the device can be controlled with an external vacuum pump connected to the device through an opening 9. To fill in the gas, an opening from the opposite side of the vessel is used, which provides a controlled gas leakage through a valve 11. To ensure that no moisture, dust, etc. enters the irradiator, a molecular sieve 10 is placed after the valve.

A working prototype has been successfully demonstrated using a discharge in air at a pressure of 5 mbar. However, as mentioned earlier, other gases may be used, which would allow embodiments to operate at different pressures.

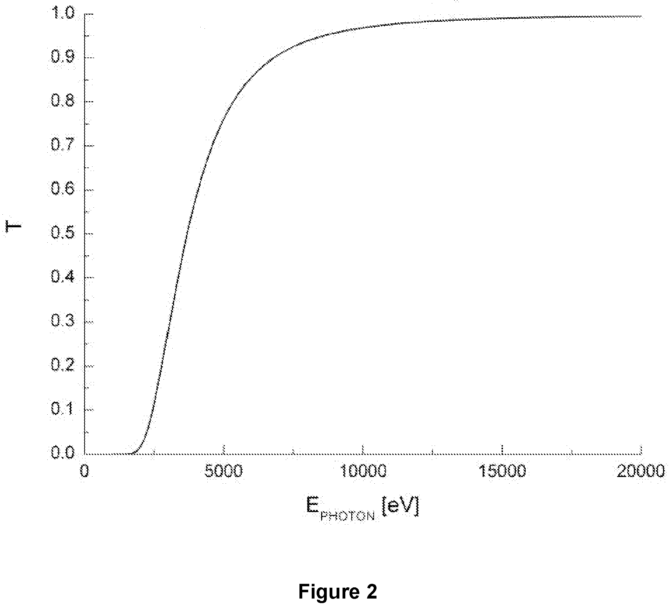

Another important part of the system is an outcoupling window 3, which forms a chamber between the anode 2 and the window 3 in which the above-mentioned gas is contained, and also encloses the cathode 1. In the presently-preferred embodiment this window 3 is made of a polyimide film, preferably Kapton™. Although other materials may be used, to date Kapton™ is the best we have found, as it demonstrates some particularly attractive features in this application, as it becomes stronger when exposed to *X-rays,* while other materials can break down or become brittle over time. Ideally the window 3 should meet several requirements: it should withstand the pressure difference and not break, have low absorption losses for *X-rays* (see transmission data in Figure 2), and should not lose its strength and transparency under the influence of intense *X-ray* irradiation. Materials other than Kapton™ can be used for the outcoupling window 3, provided they have material properties and transmission characteristics similar to those of Kapton™, or better, although at present we are not aware of any such material.

The window 3 is preferably formed of an electrically conductive material, or the inner surface of the window 3 may be covered with a thin layer of electrically conductive material. For example, a layer of conductive material such as graphite may be deposited on the inner surface of the window 3. In our presently-preferred embodiment, however, a commercially available electrically-conductive polyimide film, Kapton™ RS, is used to form the window 3. Kapton™ RS comprises a polyimide film loaded with conductive carbon. By making the window 3 electrically conductive, this enables the window 3 to be kept at the same electric potential as the grid or mesh of the cathode 1, thereby preventing an electric field from "hanging" between the cells of the cathode grid/mesh in the direction of the outcoupling window 3 (which would result in a constant flow of accelerated electrons towards the window 3, resulting in sputtering of the window material and causing it to be damaged).

The cathode 1 is mounted on, and electrically connected to, a metal support structure 15 which is kept at ground potential. Thus the cathode 1 has similar potential. The cathode support structure 15 is connected to, or integrally formed with, a housing structure 5 in which at least part of the anode 2 is mounted. The cathode support structure 15 (and the rest of the housing structure 5) is electrically isolated from the anode 2 by the insulating spacer 4. The outcoupling window 3 is also mounted on the housing structure 5, over the cathode 1. If, as in the presently-preferred embodiment, the outcoupling window 3 is electrically conductive, then the outcoupling window 3 is electrically connected to the housing structure 5 and the cathode support structure 15, so that the window 3 is at the same electric potential as the cathode 1.

The cathode support structure 15 and/or the housing structure 5 may be formed of stainless steel, or any other suitable material.

High-voltage pulses are supplied by a power supply to the irradiator via a high-voltage cable 7. The power supply is preferably a high-voltage generator with inductive energy storage. The latter is important for stable device operation, and the reasons for this are explained below.

A bulk piece of isolation material 8 matching the wave impedance of the power cable 7 with the wave impedance of the emitter head 13 serves effectively as a transformer, that reduces the reflections of the voltage pulses applied to the emitter head 13.

*Insulating (e.g. ceramic) spacer*

Although, in the presently-preferred embodiment described below, the insulating spacer 4 is made of a ceramic material (*e.g.* alumina), in alternative embodiments it can be made of other insulating materials instead.

The ceramic spacer 4 serves to insulate the anode's emitter surface 14 from the cathode support structure 15 and the metal housing 5, and simultaneously improves the operational stability of the emitter. In order to achieve this improvement in stability, we have decreased the size of the emitter surface 14 by making the opening provided by the ceramic spacer 4 slightly smaller than the opening provided by the cathode support part of the housing 15. In the example shown in Figure 1, this difference is 15 mm of additional ceramic material that projects across the surface of the anode 2. Regarding this distance by which the ceramic spacer 4 projects across the anode 2, distances shorter than 15 mm were tested, with unsatisfactory results. Distances larger than 15 mm will result in effective operation, but would reduce the area of *X-ray* emission, and hence the yield. Thus, in the presently-preferred embodiment, the distance by which the ceramic spacer 4 projects across the anode 2 is about 15 mm.

By having this ceramic spacer 4 we avoid the formation of places in the vicinity of the cathode and anode electrodes 1, 2 where the electric field strength could rise substantially. In effect, the ceramic spacer 4 *"smoothes"* the electric field distribution in the vicinity of the electrodes 1, 2. In *X-ray* sources known in the art, which have places in which the electric field strength can rise substantially, there is a substantial chance that there will be a short electric breakdown between the electrodes, resulting in arcing of the charge and a disruption of the *X-ray* generation. The result would be the erosion of the electrodes and subsequent deterioration of the inner side of the device.

As illustrated, the insulating ceramic spacer 4 preferably also extends downwards, between the housing structure 5 and the sides of the anode 2, as well as projecting across the surface of the anode 2. Thus the housing structure 5 is electrically isolated from the anode 2.

The thickness of the ceramic spacer 4 is preferably about 3 mm, as illustrated. The ceramic spacer 4 is preferably fitted in contact with the upper surface of the anode 2, and in contact with the underside of the cathode support structure 15 and the inner surface of at least part of the housing 5.

The width of the *X-ray* emitting surface 14 of the anode 2 exposed between opposing edges of the ceramic spacer 4 is preferably in the range of about 3 cm to about 10 cm. The *X-ray* beam produced from the exposed *X-ray* emitting surface 14 is homogeneous and well-directed.

As mentioned above, although the spacer 4 of the presently-preferred embodiment is made of a ceramic material *(e.g.* alumina), in alternative embodiments other insulating materials can be used instead.

Our experience shows that in the case of a regular design of the electrode, when there is no protective insulating spacer 4 (not necessarily ceramics), once in every 10,000 pulses a spark due to discharge instabilities might occur. At power levels higher than several kW, more thermally stable materials would be used; however, not a material such as Teflon™, as it is too isolating of the charge. Given that our device can operate at a repetition rate of up to 20 kHz, this effectively would mean that without these protective means the device might be suited for short-term scientific research but would be completely unfit for routine industrial operation. However, the protective insulating spacer 4 enables the present X-ray generator to be employed in long-term continuous operation, such as on a production line in a manufacturing or packaging facility.

Another important feature of our system is a combination of two protective means. One is the above-mentioned specially shaped ceramic spacer 4, and the other is the use of a power supply based not on capacitive energy storage but on inductive energy storage. The difference here occurs due to the following effects:

In a capacitive storage device if an accidental breakdown occurs, it is in no way affected by the power supply itself, and can develop a full-blown electric spark that would damage the surface of the electrodes and the device itself. However, by employing an inductive energy storage power supply, rising current results in a voltage rise on the inductors, thus effectively reducing the voltage applied to a spark. In effect, it functions as a self-damping limiter. Together with the ceramic protector 4, this substantially improves the operational stability and longevity of the device.

There is also an ancillary but beneficial by-product from the present system, namely *UV* radiation generation within the chamber between the cathode 1 and anode 2, which ensures a stable and sterile environment within the *X-ray* device.

*Summary of components in the apparatus of Figure 1, with example specifications*

1. Cathode grid (preferably copper) having a geometric transparency of about 70-80%
2. Anode (preferably copper, or iron covered with copper)
3. Output window *(e.g.* Kapton™ 200RS100)
4. Insulating spacer *(e.g.* alumina ceramic)
5. Housing *(e.g.* stainless steel) at ground potential
6. Cooling pipes (suitable cooling liquids are, for example, transformer oil or silicone oil)
7. High voltage cable
8. Isolation matching the wave impedance of the cable with the emitter head
9. Port for pumping gases *(e.g.* dry air or nitrogen, at 3-10 mbar)
10. Cartridge with molecular sieve *(e.g. X13)*
11. Regulated leakage valve
12. *X-ray* generator
13. Emitter head
14. Emitter surface
15. Cathode support structure *(e.g.* stainless steel)

<u>Soft X-rays in the Grenz-ray region</u>

The following section provides further details on the soft *X-rays* generated by the embodiments described above, and their use in sterilisation applications.

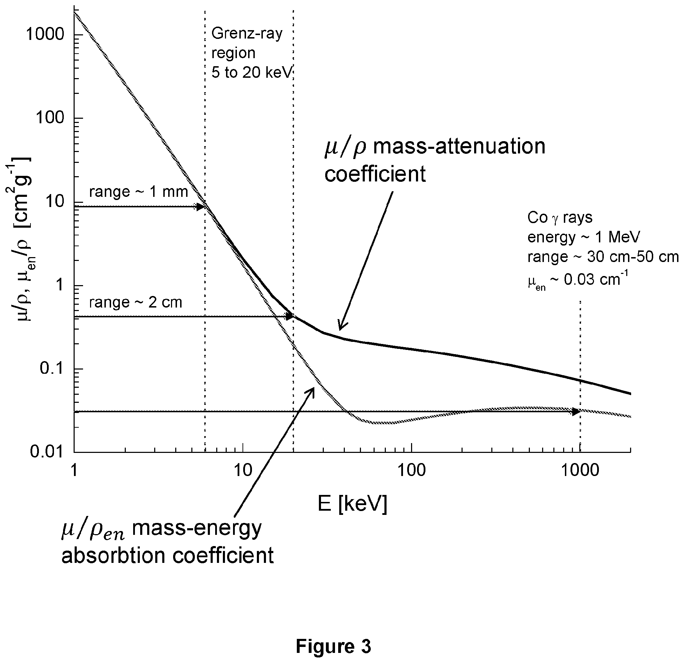

The main idea in our approach to the sterilisation of organic matter lies in the use of soft *X-rays* with quantum energies predominantly in the interval 5 to 20 keV instead of high energy gamma sterilisation using $Co^{60}$. The possible advantages and drawbacks of this approach can be seen from Figure 3 where the mass-energy absorption coefficient is presented for a typical plastic used in packaging (of density 1 g cm$^{-3}$), together with the mass-attenuation coefficient.

In the graph, the upper line is the mass-attenuation coefficient, and the lower line is the mass-energy absorption coefficient.

In the graph we have explicitly marked two different energy regions. One region corresponds to the high energy gamma rays *("hard" X-rays)* produced by $Co^{60}$ and lies near 1 MeV, while the other region (also called the Grenz-ray region) is limited within 5 to 20 keV and corresponds to *"soft" X-rays.* The lower limit of the Grenz-ray region is due to a small photon range (<1 mm) for energies less than ~5 keV. The photons with smaller energy cannot escape a traditional *X-ray* tube due to strong absorption in the vacuum window. The upper limit of the Grenz-ray region is determined by the change of mechanism by which *X-rays* interact with matter. At energy less than ~20 keV photons interact with matter predominantly via photoelectric absorption while the scattering plays minor role. At higher energies the mechanism changes to Compton scattering while photoelectric absorption is of no importance.

The main parameter which determines the effectiveness of sterilisation is the dose. The dose is the energy of *X-rays* absorbed by a unit mass of matter. It is instructive to compare the doses produced by *X-rays* with different energies. The dose-rate, produced by the flux F [ph cm$^{-2}$s$^{-1}$] of photons having energy E, is equal to the product

$$\mathrm{FE}\left(\frac{\mu_{en}}{\rho}\right)$$ . Suppose we generate equal fluxes of photons with different energies. Then the photons with different energies $E_{low}$ and $E_{high}$ will have the same *"dose efficiency"* if the products $\delta_{low} = \left(\frac{\mu_{en}}{\rho}\right)_{low} E_{low}$ and $\delta_{high} =$

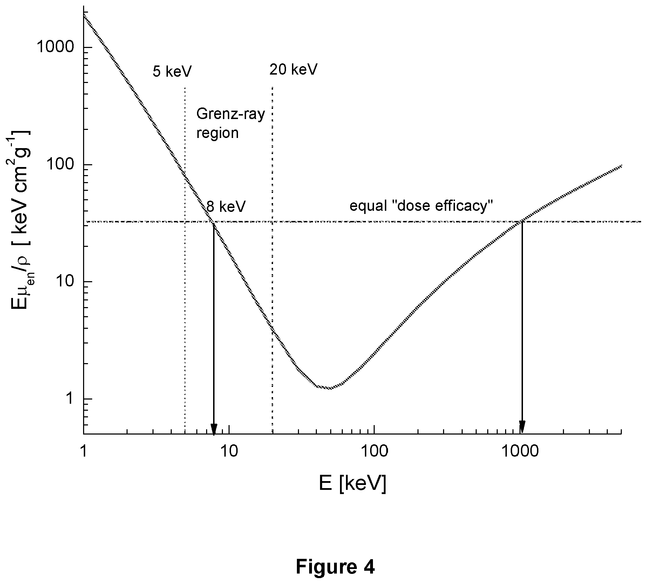

$$\left(\frac{\mu_{en}}{\rho}\right)_{high} E_{high}$$ are equal. The data for $\delta$ versus photon energy are plotted in Figure 4.

Surprisingly, there is mirror-like correspondence in dose efficiency between the low and high energy *("soft"* and *"hard" X-ray)* regions. For example, a photon with quantum energy of 8 keV has exactly the same dose efficiency as a photon with energy of 1 MeV. It is interesting to note that the photons with energy close to 50 keV are useless for the purpose of sterilisation. This is due to the deep well on the dose efficiency curve in this energy region, as shown in Figure 4.

Thus, we came to the conclusion that *X-rays* in the Grenz-ray region near 10 keV have the same dose efficiency as more energetic 1 MeV photons. The advantage of sterilisation with low energy *X-rays* becomes clearer if we compare the energy required to produce the same dose with low and high energy *X-rays.*

As the dose efficiencies are equal for energies of 8 and 1000 keV, the photon fluxes should be equal too to produce the same dose-rate. This means that the required power P=$E_{low}$F of low energy *X-rays* comprises just 8/1000 of the power of high energy gamma radiation. Suppose we generate low energy *X-rays* with efficiency of 0.8% (ratio of the output power of *X-rays* to input electric power). Then the same sterilisation effect will be achieved as with 1 MeV gamma rays if they were generated with 100% efficiency.

Sterilisation with low energy *X-rays* has a potential drawback, however. The range of low energy photons is relatively small - around 1-20 mm in plastics and water in the Grenz-ray region. It should be noted that, with plastics, there may be several layers, and the overall range of penetration of the *X-rays* in the Grenz-ray region may be more than 20 mm if the structure being irradiated is not solid plastic but contains air *(e.g.* as in foams, tubing or syringes). Of course, the photon range in atmospheric air is larger than 1 metre, even at the lower boundary of the Grenz-ray region, due to the very small density of air. It follows that there is a natural niche for low energy *X-ray* sterilisation - thin low density materials, such as medical devices, plastic packaging or blood samples, lettuce and hamburgers.

*X-ray generation by kilo-electron volts (keV) electrons*

In this section we describe a model for *X-ray* generation by electron beams impinging on metal targets and check the theoretical results against available experimental data. The most important practical result is the calculation of dose-rate at various distances from the target, which can be of use when configuring an implementation of an embodiment of an *X-ray* generator as described above. This also shows the direct advantage of our irradiation scheme for radiation sterilisation, in comparison to other sterilisation processes.

By definition, the number of X-ray photons, emitted during unit time interval within unit solid angle and unit energy interval, is as follows:

$$S_{ph}\left(\vec{\tau}, E, \vec{x}\right) = \int d\Omega_e dE_e J\left(\vec{x}, E_e, \vec{\tau}_e\right) \frac{d^2\sigma\left(\vec{\tau}_e, E_e, \vec{\tau}, E\right)}{d\Omega dE} N_{at} \qquad (1)$$

Here $J(\bar{x},\, E_e,\, \overline{\tau_e})$ is the spectral density of electrons found from the solution of transport equation, introduced above, and $\dfrac{d^2\sigma\left(\vec{\tau}_e, E_e, \vec{\tau}, E\right)}{d\Omega dE}$ is atomic field bremsstrahlung cross-section differential in photon energy and angle of emission [1].

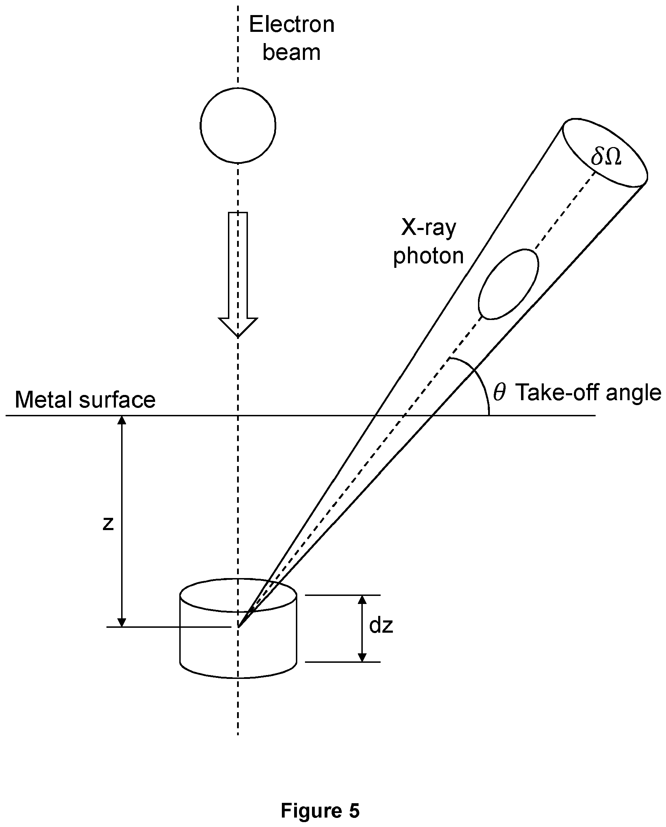

Consider the electron beam impinging normally on a metal target as shown in Figure 5. The number of photons emitted from the unit area of the target in the direction of take-off angle θ within unit energy and solid angle intervals - spectral brightness - is given by the following relation:

$$B(\vec{\tau}, E) = \int_0^\infty dz S_{ph}(z, E, \vec{\tau})\exp - \frac{\mu(E)z}{Sin(\theta)} \tag{2}$$

where $\mu(E)$ is X-ray attenuation coefficient of the target metal.

Finally, the dose-rate distribution $\dfrac{dD}{dt}$, produced by an extended *X-ray* source at the point of observation $\bar{x}$, is as follows:

$$\frac{dD}{dt} = \int d\Omega_{source} \int dE \left[ \frac{\mu_{air}(E)}{\rho_{air}} \right] EB(\vec{\tau}, E)\exp - \mu_{air}(E)L \tag{3}$$

Here the integral is taken over the solid angle at which the source is seen from the point of observation and L is the distance between the point of observation and the surface area at the source.

*Comparison of theoretical model with experimental data*

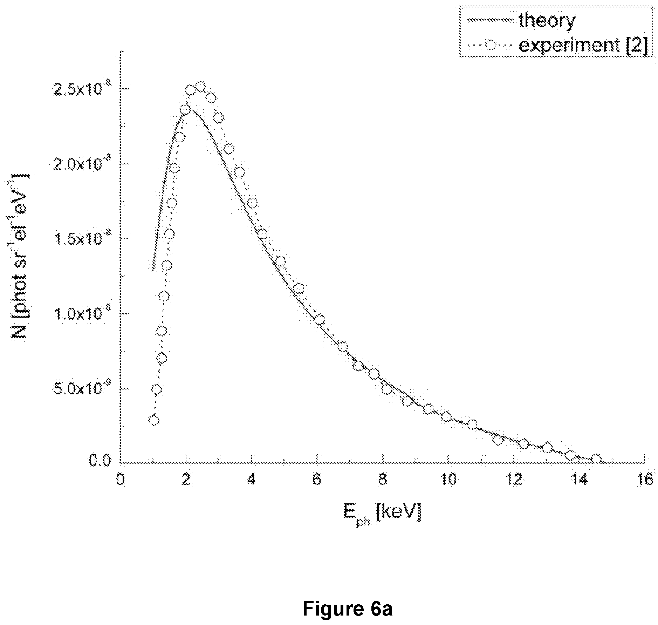

*X-ray* spectral brightness $B(\overline{\tau}, E)$ was calculated with the use of relation (2). The results of calculation are presented in Figures 6a-6c in comparison with available experimental data [2-4]. The parameters used in Figures 6a-6c are as follows:

Figure 6a: $E_{beam}$ = 15 keV, normal incidence, take-off angle = 40°.

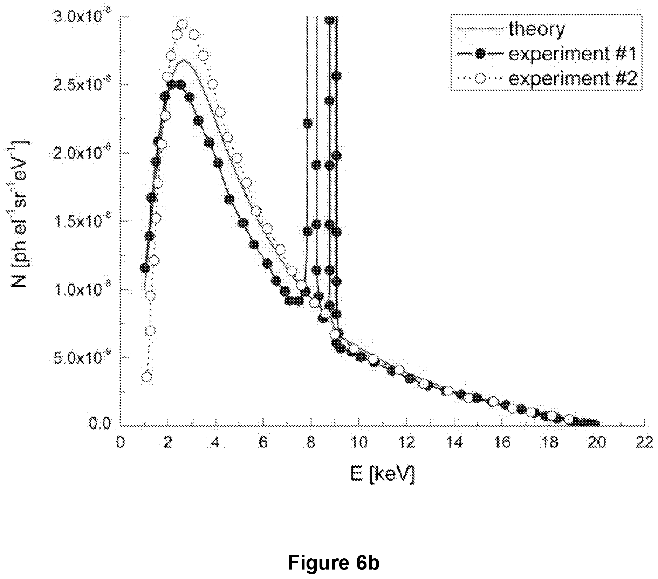

Figure 6b: $E_{beam}$ = 20 keV, normal incidence, take-off angle = 40°.

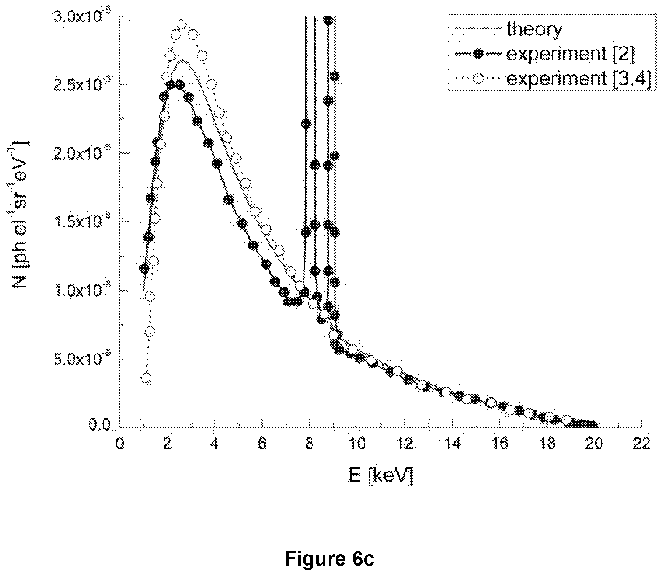

Figure 6c: $E_{beam}$ = 20 keV, normal incidence, take-off angle = 40°.

It is seen that the discrepancy between calculated and measured data is smaller than the experimental error. The theoretical curves lie right in-between the data of different authors.

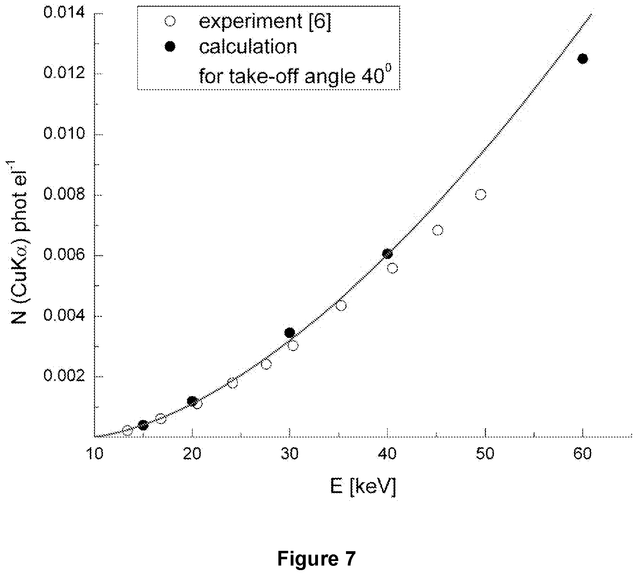

The intensity of characteristic K-radiation was calculated with the use of experimental cross-section [5]. The result of calculation is presented in Figure 7.

There is also good agreement with experiment [6]. Thus, the model developed in the course of the present work gives reliable spectra of *X-rays* generated by stopping of kilo-electron volts electron beams in metal targets and can be used for engineering of *X-ray* sterilisation sources.

*Practical example - sterilization of a plastic item using an extended X-ray source*

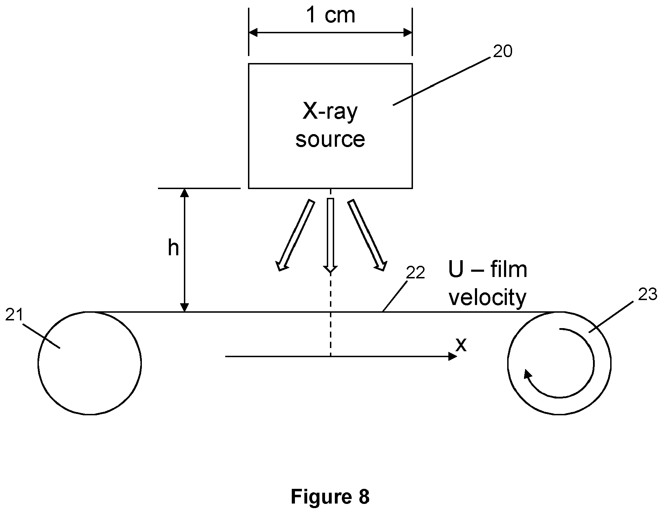

A schematic example of an *X-ray* sterilization system is shown in Figure 8. An item to be sterilized (in this case, a plastic film 22) is moved (by rollers 21 and 23) with a velocity *U* under an *X-ray* irradiator 20 comprising a rectangular *X-ray* source with a copper anode. For the purposes of this example, we take the width of the *X-ray* source to be 1 cm and its length to be 50 cm. The distance between the irradiation unit 20 and the plastic film 22 is denoted by h. The co-ordinate axis x is directed along the film.

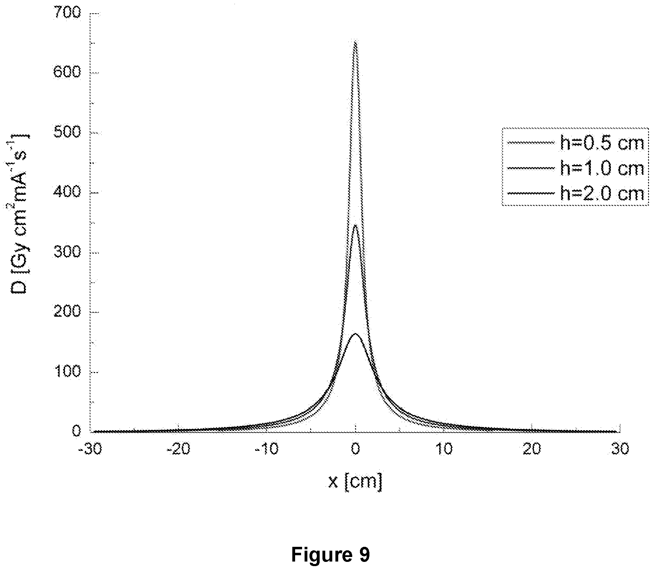

The dose-rate can be calculated with the use of equation (3). The specific dose-rate distribution at different distances h is presented in Figure 9 for the particular case of the source operating at 60 kV. The plots are based on a dose rate distribution for a rectangular source, with L=50 cm, w=1 cm, U=60 kV, and a copper anode. 'h' represents the distance to the anode. In the graph, the uppermost line refers to h=0.5 cm, the intermediate line to h=1.0 cm, and the lowermost line to h=2.0 cm.

The plots in Figure 9 present the dose received by the item to be sterilized during one second with an *X-ray* source driven by the electron beam with a current density of 1 mA cm$^{-2}$. It is important to note that peak dose-rates achieve very high values of $\sim$ 1 kGy s$^{-1}$ with very modest parameters of *X-ray* source.

To illustrate our approach, we present the calculations of the dosage delivered to a plane plastic sample during the passage of the irradiation area. The calculation of the dosage received by the item during the passage of the irradiation area is done with the use of the following relation:

$$Dosage = \frac{\int dxD}{U} J \qquad (4)$$

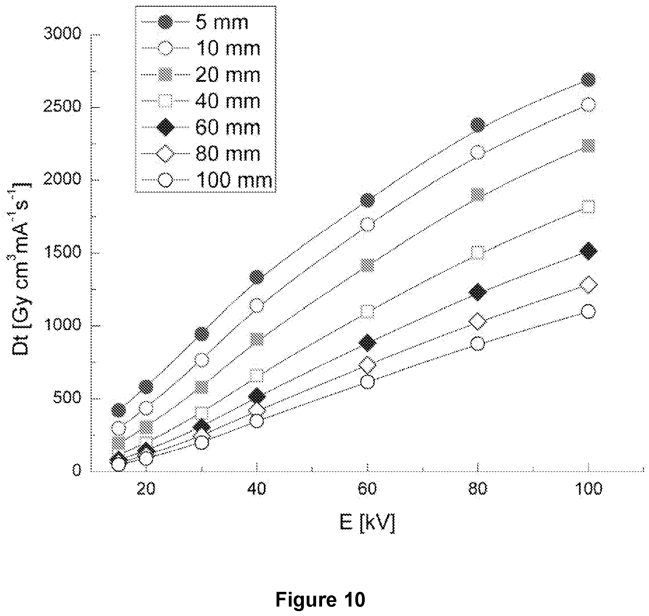

where J is the electron beam current density, *U* is the conveyor belt velocity, and dose-area integral $\int dxD$ is taken over the length of the object. The representative data for dose-area integral, calculated for different distances and voltages, are presented in Figure 10.

The electric energy *W* required for sterilisation of unit surface area of the item which is sterilized is given by the following relation:

$$W = \frac{E_{beam} Dosage_{th}}{\int dxD} \qquad (5)$$

Here $E_{beam}$ is electron beam energy in keV, $Dosage_{th}$ = 2500 Gy is the minimal dosage required for log6 reduction of bioburden, and dose-area product is in Gy cm$^2$ mA$^{-1}$s$^{-1}$. Suppose the *X-ray* source operates at 60 kV and the source-object separation is 2 cm. Then, as follows from the data in Figure 10, the required energy is:

$$W = \frac{60 \times 2500}{1300} = 115.4 \, \text{J cm}^{-2} \qquad (6)$$

*References*

[1] L. Kissel, C.A. Quarles, R.H. Pratt. Shape functions for atomic-field bremsstrahlung from electrons of kinetic energy 1-500 keV on selected neutral atoms 1 <Z<92. Atomic data and nuclear data tables 28, 381-460n (1983)

[2] Z.J. Ding, R. Shimizu, K. Obori. Monte Carlo simulation of X-ray spectra in electron probe microanalysis: Comparison of continuum with experiment. J. Appl. Phys. 76, 7180-7187 (1994)

[3] F. Salvat, J.M. Fernandez-Varea, J. Sempau et. al. Monte Carlo simulation of bremsstrahlung emission by electrons. Rad. Phys. Chem. 75, 1201-1219 (2006)

[4] E. Acosta, X. Llovet, E. Coleoni et. al. Monte Carlo simulation of X-ray emission by kilovolt electron bombardment. J. Appl. Phys. 83, 6038-6049 (1998)

[5] X. Llovet, C. Merlet, F. Salvat. Measurements of K-shell ionization cross-sections of Cr, Ni and Cu by impact of 6.5-40 keV electrons. J. Phys. B: At. Mol. Opt. Phys. 3761-3772 (2000)

[6] V. Metchnik, S.G. Tomlin. On the absolute intensity of characteristic radiation. Proc. Phys. Soc. 81, 956-964 (1963)

**Claims**

1. An *X-ray* source (12) for producing soft *X-rays* for sterilising an article, the X-ray source (12) comprising:

   a cathode (1) having an electron-emitting structure supported by a support structure (15), the electron-emitting structure being at least partially transparent to *X-rays* within a region bounded by the support structure (15);
   an anode (2) having an *X-ray* emitting surface (14) parallel to the electron-emitting structure of the cathode (1); and
   an electrically insulating spacer (4) arranged between the anode (2) and the support structure (15) of the cathode (1);

wherein the electron-emitting structure of the cathode (1) and the X-ray emitting surface (14) of the anode (2) are arranged such that, in use, the electron-emitting structure is operable to bombard the anode (2) with electrons, causing *X-rays* to be emitted from the *X-ray* emitting surface (14) and to pass through the cathode (1); wherein the *X-ray* source (12) further comprises an *X-ray* transparent window (3) on the opposite side of the cathode (1) from the anode (2), the window (3) defining a chamber between the window (3) and the anode (2); and wherein the chamber contains a gas at sub-atmospheric pressure such as to allow a discharge in gas, **characterized in that** the insulating spacer (4) projects beyond the support structure (15), across part of the anode (2), into the said region.

2. An *X-ray* source as claimed in claim 1, further comprising gas supply means in communication with the chamber; wherein the gas is an inert gas such as helium or nitrogen, or wherein the gas is air.

3. An *X-ray* source as claimed in claim 2, further comprising a molecular sieve (10) between the gas supply means and the chamber.

4. An *X-ray* source as claimed in any preceding claim, further comprising a vacuum pump in communication with the chamber.

5. An *X-ray* source as claimed in any preceding claim, wherein the window (3) is formed of an electrically conductive material or the window (3) further comprises a coating formed of an electrically conductive material; and wherein the window (3) is electrically connected to the electron-emitting structure of the cathode (1).

6. An *X-ray* source as claimed in any preceding claim, wherein the distance of projection of the insulating spacer (4) beyond the support structure (15), into the said region, is about 15 mm.

7. An *X-ray* source as claimed in any preceding claim, wherein the width of the *X-ray* emitting surface (14) not covered by the insulating spacer (4) is in the range of about 3 cm to about 10 cm.

8. An *X-ray* source as claimed in any preceding claim, wherein the thickness of the insulating spacer (4) is about 2 mm.

9. An *X-ray* source as claimed in any preceding claim, wherein the electron-emitting structure of the cathode (1) has a grid or mesh structure.

10. An *X-ray* source as claimed in any preceding claim, wherein the anode (2) further comprises cooling means.

11. An *X-ray* source as claimed in any preceding claim, wherein the support structure (15) is connected to, or integrally formed with, a housing structure (5) for the *X-ray* source; and wherein the housing structure (5) is arranged around at least part of the anode (2).

12. An *X-ray* source as claimed in claim 11, wherein the support structure (15) is electrically connected to the housing structure (5); and wherein the insulating spacer (4) extends between the housing structure (5) and the anode (2).

13. An *X-ray* source as claimed in any preceding claim, further comprising means for generating a voltage between the anode (2) and the cathode (1), wherein the means for generating a voltage comprises inductive energy storage means configured to supply high-voltage short-duration pulses to the anode (2).

14. An *X-ray* source as claimed in any preceding claim, configured to emit *X-rays* with quantum energies in the range of 5 keV to 20 keV.

15. A method of sterilising an article, the method comprising irradiating the article with *X-ray* radiation using an *X-ray* source (12) as claimed in any preceding claim, and thereby producing a sterilised article.

16. A method as claimed in claim 15, wherein the article is selected from a group comprising: a medical article; a pharmaceutical product; packaging material for a food or drink product; a plastic film; a blood sample; a foodstuff or beverage.

**Patentansprüche**

1. Röntgenstrahlenquelle (12) zur Erzeugung schwacher Röntgenstrahlen zum Sterilisieren eines Artikels, wobei die Röntgenstrahlenquelle (12) Folgendes aufweist:

   eine Kathode (1) mit einer Elektronen ausstrahlenden Struktur (14), die von einer Trägerstruktur (15) getragen wird, wobei die Elektronen ausstrahlende Struktur innerhalb einer von der Trägerstruktur (15) umgrenzten Region wenigstens teilweise für Röntgenstrahlen durchlässig ist;
   eine Anode (2) mit einer Röntgenstrahlen ausstrahlenden Oberfläche (14), die parallel zur Elektronen ausstrahlenden Struktur der Kathode (1) ist; und
   einen elektrisch isolierenden Abstandshalter (4), der zwischen der Anode (2) und der Trägerstruktur (15) der Kathode (1) angeordnet ist;
   wobei die Elektronen ausstrahlende Struktur der Kathode (1) und die Röntgenstrahlen ausstrahlende Oberfläche (14) der Anode (2) so angeordnet sind, dass die Elektronen ausstrahlende Struktur im Gebrauch dafür funktionell ist, die Anode (2) mit Elektronen zu beschießen, wodurch veranlasst wird, dass Röntgenstrahlen aus der Röntgenstrahlen ausstrahlenden Oberfläche (14) ausgestrahlt werden und durch die Kathode (1) hindurchgehen;
   wobei die Röntgenstrahlenquelle (12) ferner ein röntgenstrahlendurchlässiges Fenster (3) an der Seite der Kathode (1), die der Anode (2) entgegengesetzt ist, aufweist, wobei das Fenster (3) zwischen dem Fenster (3) und der Anode (2) eine Kammer definiert; und
   wobei die Kammer ein Gas unter subatmosphärischem Druck enthält, um ein Ablassen von Gas zuzulassen, **dadurch gekennzeichnet, dass** der isolierende Abstandshalter (4) über die Trägerstruktur (15) hinaus, über einen Teil der Anode (2) hinweg in die genannte Region vorsteht.

2. Röntgenstrahlenquelle nach Anspruch 1, die ferner eine mit der Kammer in Verbindung stehende Gaszufuhreinrichtung aufweist;
   wobei das Gas ein Inertgas wie Helium oder Stickstoff ist oder wobei das Gas Luft ist.

3. Röntgenstrahlenquelle nach Anspruch 2, die ferner ein Molekularsieb (10) zwischen der Gaszufuhreinrichtung und der Kammer aufweist.

4. Röntgenstrahlenquelle nach einem der vorhergehenden Ansprüche, die ferner eine Vakuumpumpe aufweist, die mit der Kammer in Verbindung steht.

5. Röntgenstrahlenquelle nach einem der vorhergehenden Ansprüche, wobei das Fenster (3) aus einem elektrisch leitfähigen Material gebildet ist oder das Fenster (3) ferner eine aus einem elektrisch leitfähigen Material hergestellte Beschichtung aufweist
   und wobei das Fenster (3) elektrisch mit der Elektronen ausstrahlenden Struktur der Kathode (1) verbunden ist.

6. Röntgenstrahlenquelle nach einem der vorhergehenden Ansprüche, wobei die Entfernung des Überstands des isolierenden Abstandshalters (4) über die Trägerstruktur (15) hinaus in die genannte Region etwa 15 mm beträgt.

7. Röntgenstrahlenquelle nach einem der vorhergehenden Ansprüche, wobei die Breite der vom isolierenden Abstandshalter (4) nicht bedeckten Röntgenstrahlen ausstrahlenden Oberfläche (14) im Bereich von etwa 3 cm bis etwa 10 cm ist.

8. Röntgenstrahlenquelle nach einem der vorhergehenden Ansprüche, wobei die Dicke des isolierenden Abstandshalters (4) etwa 2 mm beträgt.

9. Röntgenstrahlenquelle nach einem der vorhergehenden Ansprüche, wobei die Elektronen ausstrahlende Struktur der Kathode (1) eine Gitter- oder Maschenstruktur hat.

10. Röntgenstrahlenquelle nach einem der vorhergehenden Ansprüche, wobei die Anode (2) ferner eine Kühleinrichtung aufweist.

11. Röntgenstrahlenquelle nach einem der vorhergehenden Ansprüche, wobei die Trägerstruktur (15) mit einer Gehäusestruktur (5) für die Röntgenstrahlenquelle verbunden oder einstückig ausgebildet ist und
    wobei die Gehäusestruktur (5) um wenigstens einen Teil der Anode (2) angeordnet ist.

**12.** Röntgenstrahlenquelle nach Anspruch 11, wobei die Trägerstruktur (15) elektrisch mit der Gehäusestruktur (5) verbunden ist und
wobei der isolierende Abstandshalter (4) zwischen der Gehäusestruktur (5) und der Anode (2) verläuft.

**13.** Röntgenstrahlenquelle nach einem der vorhergehenden Ansprüche, die ferner ein Mittel zum Erzeugen einer Spannung zwischen der Anode (2) und der Kathode (1) aufweist, wobei das Mittel zum Erzeugen einer Spannung eine induktive Energiespeichereinrichtung umfasst, die zum Anlegen von Hochspannungspulsen kurzer Dauer an die Anode (2) konfiguriert ist.

**14.** Röntgenstrahlenquelle nach einem der vorhergehenden Ansprüche, die zum Ausstrahlen von Röntgenstrahlen mit Quantenenergien im Bereich von 5 keV bis 20 keV konfiguriert ist.

**15.** Verfahren zum Sterilisieren eines Artikels, wobei das Verfahren das Bestrahlen des Artikels mit Röntgenstrahlung unter Verwendung einer Röntgenstrahlenquelle (12) nach einem der vorhergehenden Ansprüche und dadurch Erzeugen eines sterilisierten Artikels umfasst.

**16.** Verfahren nach Anspruch 15, wobei der Artikel aus einer Folgendes umfassenden Gruppe ausgewählt wird: einen medizinischen Artikel, ein pharmazeutisches Produkt, Verpackungsmaterial für ein Nahrungsmittel- oder Getränkeerzeugnis, eine Kunststofffolie, eine Blutprobe, ein Nahrungsmittel oder ein Getränk.

**Revendications**

**1.** Source de rayons X (12) pour la production de rayons X à faible énergie pour stériliser un article, la source de rayons X (12) comprenant :

une cathode (1) comportant une structure d'émission d'électrons supportée par une structure de support (15), la structure d'émission d'électrons étant au moins partiellement transparente aux rayons X dans une région délimitée par la structure de support (15) ;
une anode (2) comportant une surface d'émission de rayons X (14) parallèle à la structure d'émission d'électrons de la cathode (1) ; et
un séparateur électriquement isolant (4) disposé entre l'anode (2) et la structure de support (15) de la cathode (1) ;
dans laquelle la structure d'émission d'électrons de la cathode (1) et la surface d'émission de rayons X (14) de l'anode (2) sont disposées de telle sorte qu'en cours d'utilisation, la structure d'émission d'électrons puisse être utilisée pour bombarder l'anode (2) avec des électrons, en provoquant l'émission de rayons X à partir de la surface d'émission de rayons X (14) et leur passage à travers la cathode (1) ;
la source de rayons X (12) comprenant en outre une fenêtre transparente aux rayons X (3) du côté de la cathode (1) opposé à l'anode (2), la fenêtre (3) délimitant une chambre entre la fenêtre (3) et l'anode (2) ; et
dans laquelle la chambre contient un gaz à une pression sub-atmosphérique de façon à permettre une décharge dans le gaz, **caractérisée en ce que** le séparateur isolant (4) s'avance en saillie au-delà de la structure de support (15), à travers une partie de l'anode (2), dans ladite région.

**2.** Source de rayons X selon la revendication 1, comprenant en outre un moyen d'alimentation en gaz en communication avec la chambre ;
dans laquelle le gaz est un gaz inerte tel que l'hélium ou l'azote, ou dans laquelle le gaz est l'air.

**3.** Source de rayons X selon la revendication 2, comprenant en outre un tamis moléculaire (10) entre le moyen d'alimentation en gaz et la chambre.

**4.** Source de rayons X selon l'une quelconque des revendications précédentes, comprenant en outre une pompe à vide en communication avec la chambre.

**5.** Source de rayons X selon l'une quelconque des revendications précédentes, dans laquelle la fenêtre (3) est constituée d'un matériau électriquement conducteur ou la fenêtre (3) comprend en outre un revêtement constitué d'un matériau électriquement conducteur ;
et dans laquelle la fenêtre (3) est électriquement connectée à la structure d'émission d'électrons de la cathode (1).

**6.** Source de rayons X selon l'une quelconque des revendications précédentes, dans laquelle la distance sur laquelle le séparateur isolant (4) s'avance en saillie au-delà de la structure de support (15), dans ladite région, est d'environ 15 mm.

7. Source de rayons X selon l'une quelconque des revendications précédentes, dans laquelle la largeur de la surface d'émission de rayons X (14) non couverte par le séparateur isolant (4) est d'environ 3 cm à environ 10 cm.

8. Source de rayons X selon l'une quelconque des revendications précédentes, dans laquelle l'épaisseur du séparateur isolant (4) est d'environ 2 mm.

9. Source de rayons X selon l'une quelconque des revendications précédentes, dans laquelle la structure d'émission d'électrons de la cathode (1) a une structure de type grille ou maille.

10. Source de rayons X selon l'une quelconque des revendications précédentes, dans laquelle l'anode (2) comprend en outre un moyen de refroidissement.

11. Source de rayons X selon l'une quelconque des revendications précédentes, dans laquelle la structure de support (15) est connectée à, ou formée en une seule pièce avec, une structure de logement (5) pour la source de rayons X ; et dans laquelle la structure de logement (5) est disposée autour d'au moins une partie de l'anode (2).

12. Source de rayons X selon la revendication 11, dans laquelle la structure de support (15) est électriquement connectée à la structure de logement (5) ; et dans laquelle le séparateur isolant (4) s'étend entre la structure de logement (5) et l'anode (2).

13. Source de rayons X selon l'une quelconque des revendications précédentes, comprenant en outre un moyen de génération d'une tension entre l'anode (2) et la cathode (1), dans laquelle le moyen de génération d'une tension comprend un moyen de stockage d'énergie inductive configuré pour apporter des impulsions à haute tension de courte durée à l'anode (2).

14. Source de rayons X selon l'une quelconque des revendications précédentes, configurée pour émettre des rayons X avec un quantum d'énergie de 5 keV à 20 keV.

15. Procédé de stérilisation d'un article, le procédé comprenant une irradiation de l'article avec un rayonnement sous forme de rayons X en utilisant une source de rayons X (12) selon l'une quelconque des revendications précédentes, et en produisant de ce fait un article stérilisé.

16. Procédé selon la revendication 15, dans lequel l'article est sélectionné dans un groupe comprenant un article médical, un produit pharmaceutique, un matériau d'emballage pour un aliment ou une boisson, un film de plastique, un échantillon de sang, et un produit alimentaire ou une boisson.

**Figure 1**

**Figure 2**

**Figure 3**

**Figure 4**

**Figure 5**

**Figure 6a**

**Figure 6b**

**Figure 6c**

**Figure 7**

**Figure 8**

**Figure 9**

**Figure 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2444310 A

- EP 1058286 A1

### Non-patent literature cited in the description

- **L. KISSEL ; C.A. QUARLES ; R.H. PRATT.** Shape functions for atomic-field bremsstrahlung from electrons of kinetic energy 1-500 keV on selected neutral atoms 1 <Z<92. *Atomic data and nuclear data tables,* 1983, vol. 28, 381-460n
- **Z.J. DING ; R. SHIMIZU ; K. OBORI.** Monte Carlo simulation of X-ray spectra in electron probe microanalysis: Comparison of continuum with experiment. *J. Appl. Phys.,* 1994, vol. 76, 7180-7187
- **F. SALVAT ; J.M. FERNANDEZ-VAREA ; J. SEMPAU.** Monte Carlo simulation of bremsstrahlung emission by electrons. *Rad. Phys. Chem.,* 2006, vol. 75, 1201-1219

- **E. ACOSTA ; X. LLOVET ; E. COLEONI.** Monte Carlo simulation of X-ray emission by kilovolt electron bombardment. *J. Appl. Phys.,* 1998, vol. 83, 6038-6049
- **X. LLOVET ; C. MERLET ; F. SALVAT.** Measurements of K-shell ionization cross-sections of Cr, Ni and Cu by impact of 6.5-40 keV electrons. *J. Phys. B: At. Mol. Opt. Phys.,* 2000, 3761-3772
- **V. METCHNIK ; S.G. TOMLIN.** On the absolute intensity of characteristic radiation. *Proc. Phys. Soc.,* 1963, vol. 81, 956-964